# EUROPEAN PATENT APPLICATION

(11) **EP 1 396 547 A1**
(43) Date of publication of application: **10.03.2004**
(21) Application number: 02078644.8
(22) Date of filing: 05.09.2002
(51) Int. Cl.: C12P 31/00, A61K 35/12, A61K 31/557, C07K 16/44, C12Q 1/00, G01N 33/50, C07K 14/705, A61K 39/395, A61P 35/00

(54) **Arachidonic acid metabolites and modulation of mitosis and apoptosis of cells**

(71) Applicant: Universitair Medisch Centrum Utrecht, 3584 CX Utrecht (NL); Universiteit Utrecht, 3584 CS Utrecht (NL)
(72) Inventor: Snoek, Gerry Tiny, 1015 AB Amsterdam (NL); Wirtz, Karel Willem Antoon, 3972 GD Driebergen (NL); Van Tiel, Claudia Marianne, 3734 EN Den Dolder (NL)
(74) Representative: Prins, Adrianus Willem

(57) **Abstract**

The invention concerns a new and hereto unknown characteristic of an isolated or recombinant arachidonic acid metabolite, or a functional equivalent thereof, extractable in the organic phase of a supernatant of SPIα cells and capable of increasing the survival time of 60 % of isolated human neutrophilic granulocytes to at least 48 hours, capable of increasing mitotic activity of quiescent NIH3T3 cells and capable of protecting quiescent NIH3T3 and SPIβ cells against induced apoptosis. The anti-apoptotic and mitotic activity is claimed for its use in medicaments and pharmaceutical compositions inhibiting apoptosis. Blocking said anti-apoptotic and mitotic activity is claimed for use in medicaments and pharmaceutical compositions in the treatment of cancer.

## Description

The invention relates to the fields of biochemistry and cell biology, more in particular to the treatment of cancer and the inhibition of cell death.

Phosphatidylinositol transfer proteins (PI-TP α and β) have been shown to be involved in intracellular traffic and/or metabolism of phosphatidylinositol (PI) and sphingomyelin (SM) as shown by analysis of NIH3T3 cells with increased expression of PI-TPα or β (SPIα and SPIβ cells).

Phosphatidylinositol transfer proteins (PI-TPs) belong to a family of highly conserved proteins that can catalyze the transfer of either phosphatidylinositol (PI), phosphatidylcholine (PC) or sphingomyelin (SM) between membranes. In mammalian tissues at least two low molecular weight isoforms are identified: PI-TPα, localized in the nucleus and cytosol is able to bind and transfer PI and PC and PI-TPβ, associated with the Golgi membranes that is able to transfer PI, PC and SM (Wirtz 1997). Information on the possible cellular function of the proteins has been obtained from experiments with permeabilized cells, with composed *in vitro* systems of isolated (Golgi) membranes and with cells where the expression of the proteins has been manipulated (Cockcroft 2001). The results have not yet led to a unifying hypothesis on the cellular function of both proteins. In permeabilized, cytosol-depleted cells both isoforms restored GTPγS-stimulated protein secretion as well as phospholipase C and/or PI-3-kinase mediated inositol lipid signaling. In these assays, PI-TPα and β were shown to be equally active despite their different lipid binding properties and cellular localization. On the other hand, intact mouse fibroblast cells with increased expression of either PI-TPα or PI-TPβ demonstrated remarkable differences in lipid metabolic pathways. Cells with increased expression of PI-TPα (SPIα cells) showed an enhanced phospholipase A₂ (PLA₂)-mediated degradation of PI resulting in increased levels of lysoPI, glycerophosphorylinositol (GPI), inositol(1)P (Ins(1)P) and inositol(2)P (Ins(2)P) (Snoek 1999). This effect was not observed in cells with increased expression of PI-TPβ (SPIβ cells). However, in SPIβ cells (but not in SPIα cells) it was shown that under conditions where SM from the plasma membrane was hydrolyzed to ceramide by exogenous sphingomyelinase, PI-TPβ was able to maintain the steady-state level of SM in the plasma membrane (van Tiel 2000).

In the present invention it was found that the rate of proliferation of SPIa cells is significantly increased. The cell cycle duration decreased from 21 h for wild type cells to 13 h for SPIα cells indicating that PI-TPα is involved in the production of a mitogenic factor, either intracellularly or extracellularly. We observed that in SPIα cells a PI-specific PLA₂ is activated which as demonstrated in the present invention leads, in addition to the production of lysoPI, GPI, Ins(1)P and Ins(2)P, to the production of a significant amount of free arachidonic acid. Arachidonic acid is the main poly-unsaturated fatty acid in the production of eicosanoids that are oxygenated C₁₈, C₂₀ and C₂₂ carbon fatty acids. Arachidonic acid can be converted into prostaglandins, leukotrienes or thromboxanes by cyclooxygenases or lipoxygenases.

It is demonstrated herein that SPIα cells display a highly increased rate of proliferation as a result of the production of PI-specific PLA₂ arachidonic acid metabolites, which are at least in part secreted into the medium. This release of mitogenic arachidonic acid metabolites is further enhanced upon stimulation of the SPIα cells with the calcium ionophore A23187.

SPIα cells were shown to be highly resistant to UV- or TNFα-induced apoptosis and, surprisingly, the conditioned medium of SPIα cells was able to establish the survival of wtNIH3T3 cells, SPIβ cells and human neutrophils upon induction of apoptosis.

Analysis of the expression of COX-2 by Western blotting showed an increased expression of COX-2 in SPIα cells. Specific inhibition of COX-2 with NS398 abolished the release of the mitogenic activity by SPIα cells. These results show that PI-TPα is involved in the production of an arachidonic acid metabolite that is produced by COX-2 and that demonstrates mitogenic and anti-apoptotic activity. Therefore, this application shows that a new characteristic, namely the inhibition of apoptosis and the increase of mitotic rate, can be attributed to a supernatant (i.e. conditioned medium), or part thereof, isolated from a cell having an active COX system, said cell over-expressing PI-TPα. Said cell is preferably an animal cell, more preferably, a mammalian cell. In a particularly preferred embodiment said cell is a murine or a primate cell, more preferably a mouse cell or a human cell. In addition, we teach in this application that, for example, from isolated neutrophilic granulocytes, the majority of which normally perishes due to programmed cell death or apoptosis within 12 to 24 hours, at least 50% can be maintained for at least 48 hours, if contacted with a sufficient amount of conditioned medium that was collected after said SPIα cells had been cultured in it.

As used herein the term "arachidonic acid metabolite"refers to a product originating directly or indirectly from arachidonic acid. The term also relates to the identical compound when produced at least in part synthetically. In a preferred embodiment said metabolite is derived from arachidonic acid by enzymatic processing preferably by a COX enzyme.

Of course, a functional equivalent of an arachidonic acid metabolite is also included in the invention. A functional equivalent is meant to include any substance that has the same effect in kind if not in amount, as said arachidonic acid metabolite. Said arachidonic metabolite can be isolated from said supernatant according to standard methods that are known in the art. Said standard methods may comprise for example: labelling of cells with ¹⁴C arachidonic acid, extraction of fatty acids from said supernatant with an organic solvent, evaporation of the solvent under N₂, spotting the residue on a silica gel thin layer chromatography plate and monitoring the radioactivity by scanning the plate with a TLC-Linear analyser and obtaining the arachidonic metabolite from the corresponding part of the plate.

Therefore, this invention provides an isolated or recombinant arachidonic acid metabolite, or a functional equivalent thereof, extractable in the organic phase and capable of increasing the survival time of at least 50 % of isolated human neutrophilic granulocytes to at least 48 hours, capable of at least in part protecting wtNIH3T3 and SPIβ cells against UV-induced apoptosis, and capable of increasing mitotic activity of quiescent NIH3T3 cells. By identifying the arachidonic acid metabolites that are formed in the supernatant of said SPIa cells as the active substances which, as one of their activities, increase the mitotic rate of quiescent cells and inhibit apoptosis of neutrophilic granulocytes, this application teaches an isolated or recombinant arachidonic acid metabolite, or a functional equivalent thereof, obtainable from a supernatant of said SPIα cells. Said arachidonic acid metabolite can be extracted from said supernatant by making use of fatty acid characteristics. Therefore, this invention provides an arachidonic acid metabolite, or a functional equivalent thereof, extractable in the organic phase and capable of increasing the survival time of at least 50 % of isolated human neutrophilic granulocytes to at least 48 hours, capable of increasing the mitotic activity of quiescent NIH3T3 cells and capable of protecting wtNIH3T3 and SPIβ cells against UV-induced apoptosis. Said arachidonic acid metabolite may be used in cooperation with a further arachidonic acid metabolite, this application, therefore, further provides a composition comprising an arachidonic acid metabolite, or a functional equivalent thereof, of the invention said composition further comprising a further arachidonic acid metabolite Said further arachidonic metabolite may be more abundant in conditioned medium of SPIα cells, therefore this application also teaches a composition comprising an arachidonic metabolite, or a functional equivalent thereof, of the invention further comprising a further arachidonic acid metabolite obtainable from a supernatant of SPIα cells. The conversion of arachidonic acid by one of the metabolizing pathways depends on the cell type. Eicosanoids are typically not stored but synthesized on demand in which the release of arachidonic acid by PLA₂ is a rate-limiting step

In relation to cell growth and cell survival, in particular the products of the two isoforms of cyclooxygenase, COX-1 and COX-2, the prostaglandins have been shown to have unique physiological roles as well as coordinated functions (reviewed by (Smith et al. 2001)). In general, COX-1 is expressed constitutively whereas COX-2 can be induced by physiological stimuli and is expressed, for instance, at sites of inflammation and carcinogenesis (Vane et al. 1998; Williams et al. 1999). Both isoforms catalyze the same reaction but are regulated by different signaling systems and show different affinity constants (Kₘ's) towards their substrate arachidonic acid (Vane et al. 1998). Growth factors, cytokynes and tumor promotors stimulate the expression of COX-2 as well as the arachidonic metabolites that are produced by the protein itself (Slice et al. 2000). It has been suggested that an increase in arachidonic acid metabolism depends on proteins that coordinate the availability of arachidonic acid towards its metabolism by COX-1 or 2. Thus, PI-TPα-mediated activation of PLA₂ as occurs in SPIα cells leads to an increased COX-1/2-mediated production and the secretion of arachidonic acid metabolite(s) that, by an autocrine pathway, are responsible for the altered growth characteristics and survival of SPIα cells.

This invention provides a method to produce and obtain said arachidonic acid metabolite comprising: Increasing PI-TPα expression in cells comprising COX, culturing said cells *in vitro*, optionally stimulating said cells with a calcium ionophore, before collecting supernatant of said cells, extracting eicosanoids from said supernatant medium, said cells or a combination thereof.

Extraction of eicosanoids is preferably performed with an organic solvent such as for example ethyl acetate or a similar solvent. Therefore this application teaches a method as described above further comprising extracting eicosanoids by ethyl acetate and/or a functional equivalent thereof. Preferably, said arachidonic metabolite is at least partially purified from other arachidonic acid metabolites. Therefore, this application teaches a method as described above, further comprising at least partially separating said arachidonic acid metabolite from prostaglandins PGF_{2α} and PGE₂. Said method will provide an arachidonic acid metabolite with the desired characteristics as described above, therefore this application teaches an arachidonic acid metabolite obtainable by said methods. Said cell supernatant and said arachidonic acid metabolite can be used for at least in part inhibiting apoptosis of a eukaryotic cell and/or increasing the mitotic rate in quiescent cells. Inhibiting apoptosis in an eukaryotic cell may lengthen the survival time of cells in a tissue that has been isolated from the body, such an organ to be transplanted or any tissue, taken from the body. Thus, the invention provides the use of a cell supernatant and/or an arachidonic acid metabolite of the invention and/or a functional equivalent thereof, or a composition of the invention, for at least in part inhibiting apoptosis of a eukaryotic cell. Apoptosis may also occur in the body, for example as a result of hypoxia or anoxia. Apoptosis is often observed after acute heart failure or after stroke. Inhibition of apoptosis in the body may prevent or at least partly diminish the adverse affects observed after apoptosis related diseases like for example occurs in patients recovering from a stroke and/or suffering from heart failure. Therefore, the invention provides the use of a cell supernatant and/or an arachidonic acid metabolite of the invention and/or a functional equivalent thereof, or a composition of the invention for the manufacture of a medicament for the treatment of apoptosis related disease. In a preferred embodiment, this invention provides a pharmaceutical composition comprising a cell supernatant and/or an arachidonic acid metabolite of the invention and/or a functional equivalent thereof, or a composition of the invention and a suitable carrier or solvent. In another embodiment, this invention provides said pharmaceutical composition for use in treating apoptosis related disease.

An apoptosis related disease is a disease in which at least some of the pathology is caused by increased apoptosis of cells in the body of the patient when compared to the same cells in a healthy individual with roughly the same age and life style. Non-limiting examples of such diseases are : Parkinson (Nagatsu 2002), Amyotrophic Lateral Sclerosis (Sathasivam et al. 2001), myocardial infarction (Sabbah et al. 1998), biliary cirrhosis (Graham et al. 1998), Alzheimer (Engidawork et al. 2001) and glaucoma (Harris et al. 1999).

Arachidonic acid metabolites play important roles in many cellular processes like thrombosis (Macchia et al. 1995), inflammation, pain and fever (Morita et al. 1999), Alzheimer's disease (Lukiw et al. 2000), cancer , cell growth and apoptosis (Avis et al. 2001). Several cancer cells (breast, colon, lung) have been shown to produce large amounts of prostaglandins (Dang et al. 2002). On the other hand, prostaglandins can also have anti-tumor activity (Ratnasinghe et al. 2001).

Inhibiting the mitotic and anti-apoptotic effects of said arachidonic acid metabolites may decrease the growth of tumor cells. Said inhibiting effect may be caused by contacting arachidonic acid metabolites with an antibody. Once an arachidonic acid metabolite is defined and isolated according to this application, it will be clear to any person skilled in the art how to produce and select an antibody against said arachidonic acid metabolite. Therefore, this invention provides an isolated or recombinant antibody capable of specifically binding an arachidonic acid metabolite or a functional part, equivalent thereof or a functional equivalent of said antibody. A functional equivalent of an antibody comprises any part, derivative or analogue of said antibody that has the same function in kind, if not in amount. With said antibody, inhibition of the action of said arachidonic acid metabolite on a cell can be performed. Further, with said arachidonic acid metabolite a cell can be defined comprising a cellular receptor for said arachidonic acid metabolite. Therefore, this application provides a method for identifying a cell carrying a functional receptor for an arachidonic acid metabolite, said receptor specifically increasing mitosis and decreasing apoptosis in said cell. The effects of said arachidonic acid metabolite on said receptor can be measured. Therefore, this application provides a method for identifying a functional receptor for an arachidonic acid metabolite, said receptor upon activation specifically increasing mitosis and decreasing apoptosis in said cell. Once said receptor is known, its amino acid sequence can be determined, and subsequently, its genetic code can be established. Thus, a cellular receptor for an arachidonic acid metabolite capable of increasing mitosis and decreasing apoptosis in a cell is provided. Inhibiting the signalling of an arachidonic acid metabolite to said receptor may decrease mitotic rate and the lifespan of a cell. In the treatment of cancer cells these are important features that help combating cancer. Therefore, an antagonist to said receptor is important for inhibiting the mitosis and lifespan of cancer cells. Thus, this invention provides an antagonist to a cellular receptor as described above, said antagonist capable of at least in part inhibiting the bioactivity of said arachidonic metabolite on its cellular receptor. In another embodiment, this application provides a method for at least in part inhibiting the mitogenic and/or anti-apoptotic signalling activity of an arachidonic acid metabolite of the invention, comprising providing an antagonist of said arachidonic acid metabolite at least in part inhibiting the bioactivity of said arachidonic acid metabolite on its cellular receptor. Said method can be optimised by also providing COX enzyme inhibitor. Thus, this invention provides said method further comprising inhibiting COX enzymes.

Said bio-availability can be decreased by providing an antagonist or by providing an antibody against said arachidonic acid metabolite. Thus, this invention also provides a method wherein said antagonist decreases the bio-availability of said arachidonic acid metabolite and/ or said antibody decreases the bio-availability of said arachidonic acid metabolite. A medicament for the treatment of cancer can be manufactured by using said antagonist, and said medicament can also be used comprising the use of a COX enzyme inhibitor. Said antagonist and said COX enzyme inhibitor can be used for a pharmaceutical composition, thus this invention also provides a pharmaceutical composition comprising said antagonist and/or a COX-inhibitor and a suitable carrier or solvent. A person skilled in the art will know how to use said pharmaceutical composition as a medicament for the treatment of cancer, therefore this invention teaches the use of said pharmaceutical composition for the preparation of a medicament for the treatment of cancer.

### EXAMPLES

### MATERIALS AND METHODS

### Materials.

Indomethacin, NS398, PGE₂-Elisa kit, Cox-1/Cox-2 antibody, ³H-thymidine, ¹⁴C-arachidonic acid,

### PI-TP constructs

The cDNA encoding mouse PI-TPα was isolated and cloned into the pBluescript vector (Geijtenbeek 1994). The PI-TPα cDNA contains a *Nco*I restriction site around the translational start codon and an *Eco*RI and *Xho*I site downstream of the translational stop codon. The *Nco*I-*Xho*I fragment was isolated (including the *Eco*RI site) and ligated into the cloning vector pUC21 in the corresponding restriction sites in order to introduce an extra *Eco*RI site upstream of the PI-TPα cDNA. The resulting *Eco*RI fragment (containing the complete coding cDNA) was cloned into the unique *Eco*RI site of the pSG5 expression vector. A construct was selected with the cDNA encoding PI-TPα in the sense direction. This construct will be denoted as pSG5-PI-TPα. The expression of PI-TPα will be regulated by the SV40 early promoter, and polyadenylation will be provided by the SV40 poly(A)-adenylation signal.

The cDNA encoding mouse PI-TPβ was isolated and cloned into the pGEX-2T vector. The PI-TPβ cDNA contains a XbaI site upstream of the translational start codon and a BamHI and SalI site downstream of the translational stop codon. The XbaI-SalI fragment (including the BamHI site) was isolated and cloned into the corresponding restriction sites of the cloning vector pBluescript SK⁻ in order to obtain a SacI site upstream of the PI-TPβ cDNA. The resulting SacI-BamHI fragment (containing the complete PI-TPβ cDNA) was cloned into the corresponding restriction sites of the pBK-CMV expression vector. This construct will be referred to as pBK-CMV-PI-TPβ. The expression of PI-TPβ will be regulated by the CMV immediate early promoter and the SV40 poly (A)-adenylation signal will provide the signal for termination of eukaryotic transcription and polyadenylation.

### Transfection

wtNIH3T3 fibroblast cells were transfected using the calcium phosphate precipitation method. Briefly, cells were seeded 5 h prior to transfection at a density of 1.3x10⁴ cells/cm² and then transfected either with 30 µg ofpSG5-PI-TPα and 10 µg of pSV2-neo to obtain SPIα cells or with 20 µg pBK-CMV-PI-TPβ to obtain SPIβ cells. Fresh medium was added 20 h after transfection and the next day the cells were reseeded at a density of 250 cells/cm². After another 24 h, G418 (0.4 mg/ml) was added for selection of G418-resistant cells. Fresh medium containing G418 was added every 4 days and resistant clones were identified after 3 weeks of growth.

### Cell culture and growth assays.

All cells were cultured in Dulbecco's modified Eagle's medium (DMEM) containing 10% newborn calf serum (NCS) and buffered with NaHCO₃ (44 mM) in a 7.5% CO₂ humidified atmosphere at 37 °C.

To determine growth rate, cells (1 x 10⁴ per well) were seeded in 24 wells plates. After 24 h the medium was replaced by 1 ml DMEM/Bic containing 0.1% NCS. After 24-48 h the medium was replaced by 250 µl conditioned medium or 250 µl DMEM/Bic/0.1% NCS supplied with extracts of the media. After 8h [³H]-thymidine (0.5 µCi/well in 50 µl DMEM/Bic/0.1% NCS) was added to the cells. After 16 h (overnight) the medium was removed, the cells were washed 4 times with phosphate buffered saline and 1 ml of methanol per well was added. The methanol was removed after 20 minutes; the cells were left to dry on air. 0.5 ml of 0.1N NaOH was added, the cells were incubated for 30 minutes at 37°C and scraped of. The cells were mixed with 4.5 ml of scintillation liquid and counted. Controls were cells incubated with 250µl of DMEM/Bic/0.1% NCS. Maximal stimulation was obtained by the addition of DMEM/Bic containing 10% NCS.

### Preparation of conditioned medium.

Cell cultures (75 or 150 cm² dishes) were grown to 80-90% confluency. The medium was replaced by 5 or 10 ml of DMEM/Bic containing 0.1% BSA (DBB). This medium was left on the cells for 24h. After removal, the medium was centrifuged (5 min at 1000 rpm) to remove floating cells. The supernatant is the crude conditioned medium.

Conditioned medium in the presence of A23187 was prepared differently in order to enable a significant decrease in the concentration of A23187 by dilution. The cells (75 or 150 cm² dishes) were washed twice with Phosphate Buffered Saline without Ca²⁺ and Mg²⁺ (PBSO) and incubated in 8 mM EGTA in PBS0 at 37°C for 5-10 minutes. The cells were resuspended, centrifuged for 5 minutes at 100 rpm. The cell pellet was resuspended in 250-500 µl of DMEM/Bic/0.1% BSA with or without A23187. The suspension was rotated for 5 minutes at 37°C, the cells were removed by centrifugation and the supernatant, i.e. the conditioned medium, was diluted to 5 or 10 ml.

Under standard conditions, quiescent cells were incubated with conditioned medium that was derived from an identical surface of cells, *i.e.* each 2 cm² well of a 24-wells dish was incubated with the amount of conditioned medium that was conditioned for 24 h by 2 cm² of cell.

### Induction of apoptosis by UV-irradiation

For UVC treatment cells were grown in a 6- or 12 well dish. Before UVC treatment the cells were incubated for 4 h in bicarbonate-buffered DMEM containing 0.1% bovine serum albumin (DBB). The medium was removed and UV treatment was performed in a Stratalinker (Stratagene) with the indicated doses (standard: 200 J/m²). After treatment with UV light, 1 ml of DBB was added and cells were incubated at 37°C. At the indicated times cell death was morphologically scored as the percentage of cells that are in the process of blebbing, that have a condensed nucleus or that have lysed. Human neutrophils were incubated with DBB or conditioned medium of SPIα cells and apoptosis was assessed after 24 and 48 h.

### Labeling cells with ¹⁴C arachidonic acid; extraction and TLC of cells and conditioned medium.

Cells were grown to 60% confluency. The cells were labeled for 24 h with [¹⁴C]-arachidonic acid (0.1 µCi per well in a 6-wells plate in 1 ml of DMEM/Bic containing 10% NCS). To analyze labeled arachidonic acid metabolites in conditioned medium, the label medium was removed after 24 h and replaced by DMEM/Bic with 0.1% BSA. After 24 h this medium was collected, centrifuged to remove floating cells. Arachidonic acid metabolites in the conditioned medium were extracted and separated as described by (Tai et al. 1979). Shortly, 0.03 ml of 12M formic acid was added per ml of conditioned medium. The mixture was extracted with two 3 ml portions of ethyl acetate. The combined extracts were evaporated under N₂. The residue was taken up in acetone and quantitatively spotted on a silicagel TLC plate. The TLC plate was developed in the organic phase of ethyl acetate/acetic acid/iso-octane/water (11:2:5:10, by vol.) as the solvent system. Radioactivity was monitored by scanning the plate with a Berthold Tracemaster 20 Automatic TLC-Linear analyzer.

### FACS analysis of fragmented DNA.

Cells were grown in 9 cm² dishes to 80% density. Cells were treated as described above (preincubation with conditioned medium for 4h), UV treatment was performed and 1.5 ml of DBB was added to the cultures. Cells were kept at 37°C. After 2 h the medium was collected, cultures were washed with 1 ml of PBS0, the PBS0 was combined with the medium and centrifuged in order to collect floating cells. Cell cultures were incubated with 1 ml of 8 mM EGTA in PBS0 for 5 min, resuspended and centrifuged for 5 min at 800 rpm at 4°C. Both cell pellets were combined and resuspended in 100 µl PBS0 supplemented with 0.5% polyvinyl alcohol (PVA), vortexed and kept on ice for 30 min. 900 µl of 70%ethanol in PBS (0°C) was layered carefully on top of the PBS-PVA. The sample was mixed and stored at -20°C. For FACS analysis nuclei were extracted and stained. The cell suspension was centrifuged for 3 min at 2200 rpm at 4°C. The supernatant was removed, the pellet was resuspended in 1 ml of PBS and again centrifuged for 3 min at 2200 rpm. The pellet was resuspended in 1 ml of a mixture of 50% PBS and 50% extraction buffer (0.2 M Na₂HPO₄, 4 mM citric acid, pH 7.8). The suspension was kept for 10 min at RT. Nuclei were collected by centrifugation (3 min 2200 rpm), the pellet was resuspended in 1 ml of PBS containing propidium iodide (50 µg/ml) and RNase (50 µg/ml) and the suspension was incubated in the dark for at least 30 min. Before FACS analysis, the mixture was filtered through a 70 µm cell strainer (Falcon) in order to remove clustered nuclei and other debris. Stained nuclei were analyzed in a FACS analyzer and the percentage of fragmented DNA was determined.

### Analysis of COX-1 and COX -2 by electrophoresis and Western blotting.

Cells were grown in 9 cm² dishes to 80-90% density. Cells were washed twice with PBS0 and conditioned medium was added. Cells were harvested after 10, 30, 60, 120, 180 and 240 min. To harvest the cells, the medium was removed, cells were washed twice with PBS0 and the dishes were frozen. After thawing the cells were incubated with 150 ml of buffer containing 0.1% Nonidet P40, 40 mM β-glycerophosphate, 1 mM Na₃VO₄, 50 mM NaF, 10 µg/ml aprotinin, 2 mM PMSF for 15 min at room temperature, scraped off and put on ice. The cell lysate was centrifuged for 5 min at 14,000 rpm at 4°C and the supernatant was used to determine protein content. Equal amounts of protein of all samples were prepared for gelelectroforesis. 25 µg of supernatant protein was loaded on an SDS-PAGE gel and gel electrophoresis was performed. The proteins were electrophoretically transferred to a nitrocellulose sheet by wet-blotting for 75 min at 100 V. Quantification of bands on an immunoblot or film was performed by scanning with a BioRad GS 700 imaging densitometer equipped with an integrating program.

### RESULTS.

### Mitogenic activity in medium that has been conditioned by SPIα and wtNIH3T3 cells.

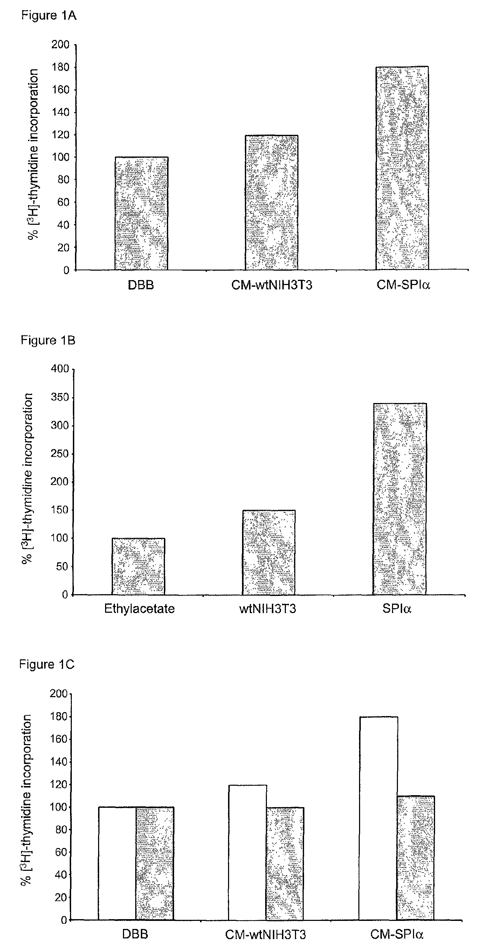

It was shown that SPIα cells demonstrate a highly increased proliferation rate as well as an increased cell density at saturation (Table 1). Despite the high level of homology between PI-TPα and β (77% identity and 94% similarity), SPIβ cells display opposite characteristics: a decreased proliferation rate and a decreased saturation density. Because of the highly increased proliferation rate of SPIα we investigated whether a growth factor (either a polypeptide growth factor or a bio-active lipid metabolite) is produced by the cells. Medium (DMEM/Bic + 0.1% BSA) that has been conditioned by exponentially growing cultures of wtNIH3T3 or SPIα cells (*i.e.*: 'conditioned medium'), was added to serum-starved (24-48h) wtNIH3T3 cells. The volume of medium that has been conditioned by 2 cm² of cells was added to each well of a 24 wells (2 cm² each) plate. DNA synthesis was determined as incorporation of [³H]-thymidine. The conditioned medium of SPIα cells increased the incorporation with 80% (Figure 1A). The conditioned medium of SPIβ cells (not shown) had the same effect on cell growth as the medium of wtNIH3T3 cells (an increase of 20%). Post-nuclear cell lysates did not affect the growth rate of quiescent wtNIH3T3 cells (not shown). This indicates that SPIα cells produce a mitogenic factor that is secreted into the medium.

Based on former results (Snoek et al. 1999) it can be assumed that PI-specific PLA₂, regulated by PI-TPα, is involved in the production of the mitogenic factor. The observed production of lysoPI coincides most likely with the production of arachidonic acid which subsequently is processed by cyclooxygenases or lipoxygenases into prostaglandins or leukotrienes to be secreted by the cells into the medium. To isolate prostaglandins and leukotrienes from the conditioned medium, we used a neutral extraction procedure (Tai et al. 1979). As shown in Figure 1B, the organic phases of extracted conditioned medium of wtNIH3T3 cells slightly increased the incorporation of [³H]-thymidine in quiescent wtNIH3T3 cells when compared to the blank. The activity of the organic phase of extracted conditioned medium of SPIβ cells is comparable to that of wtNIH3T3. However, the extract of the conditioned medium of SPIα cells stimulated the growth of quiescent wtNIH3T3 cells 3.5-fold. The amount of extract that was added to 2 cm² of cells was derived from medium conditioned by 2 cm² of cells and thereby the concentration of mitogenic factor that is added to the cells in figure 1A and 1B, is comparable. The aqueous phase of the extraction did not contain mitogenic activity (not shown). When the medium was conditioned in the continuous presence of 10 µM indomethacin, a non-selective inhibitor of COX-1 and COX-2 or 50 µM NS398, a selective inhibitor of COX-2, the organic phase after extraction was no longer mitogenic for quiescent wtNIH3T3 cells (Fig 1C). Treatment of the cells with Ca²⁺-ionophore A23187 resulted in a rapid release of the mitogenic arachidonic acid metabolites in the SPIα cells. This was not observed with the NIH3T3 or SPIβ cells (data not shown). These results further emphasize that cells with increased expression of PI-TPα demonstrate an enhanced metabolism of arachidonic acid presumably derived from PLA₂-mediated degradation of PI.

### Isolation and identification of the mitogenic factor that is produced by SPIα cells.

The finding that indomethacin and NS398 block the production of the mitogenic factor strongly suggests the presence of an arachidonic acid metabolite in the conditioned medium of SPIα- that is able to stimulate cell proliferation. In order to identify the nature of the secreted mitogenic metabolite, we labelled the cells for 24 h with [¹⁴C]-arachidonic acid, then removed the labelling medium, added fresh serum-free medium containing 0.1% BSA, and collected the medium after 24h (this is identical to the conditioned medium in the growth assays). The extracted mediums were separated by TLC and the radioactive peaks were detected by scanning the TLC plate.

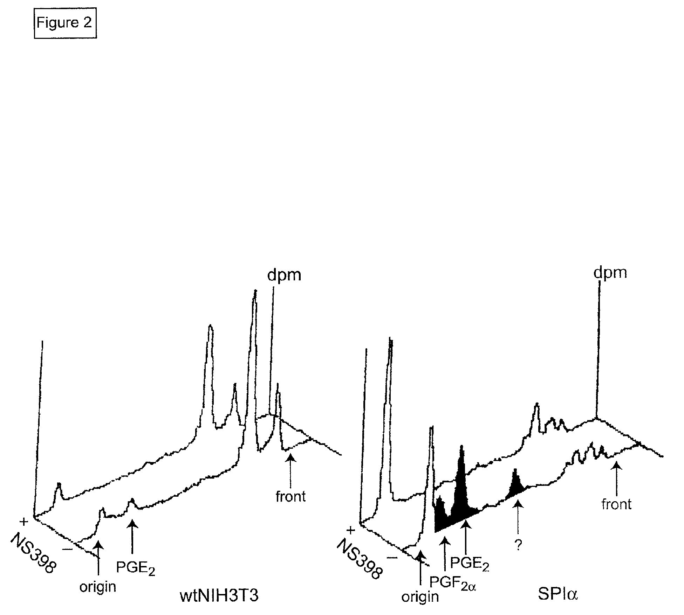

The conditioned medium of SPIα cells contained three additional peaks when compared to conditioned medium of wtNIH3T3 cells (Fig 2). When the conditioned medium was prepared in the presence of 10µM indomethacin or 50 µM NS398, the three extra peaks in the conditioned medium of SPIα cells were absent (Fig 2). Using pure prostaglandins as a standard it could be shown that two of the arachidonic metabolites in SPIα-conditioned medium co-eluted with PGE₂ and PGF_{2α} respectively. The PGE₂ concentration in the conditioned medium of wtNIH3T3 and SPIα cells was determined by Enzyme-Linked ImmunoSorbent Assay using an ELISA kit and it was shown that SPIα produces 5 times more PGE₂ when compared to wtNIH3T3 The mitogenic activity of PGE₂ and PGF_{2α} on quiescent wtNHI3T3 cells was investigated. It was shown that both prostaglandins, either separate or combined, were unable to stimulate the growth of quiescent wtNIH3T3 cells in the concentration range present in the conditioned medium of SPIα cells (not shown). It still remains to be determined at what position(s) the active arachidonic acid metabolite(s) collect on the TLC-plate.

In summary we have found that at least three arachidonic acid metabolites are specifically produced by SPIα cells, and that the production of these metabolites is inhibited by indomethacin and NS398. Furthermore two of the metabolites co-elute with either PGE₂ or PGF_{2α} both of which are not able to stimulate the proliferation of quiescent wtNIH3T3 cells. Also the medium conditioned in the absence of indomethacin is mitogenic and in the presence of indomethacin is not mitogenic. These findings indicate that PI-TPα is involved in the production of a mitogenic arachidonic acid metabolite different from PGE₂ and/or PGF_{2α}, thereby, stimulating proliferation by an autocrine pathway.

### Cell survival of wtNIH3T3, SPIα and SPIβ cells.

Factors that stimulate cell growth often also demonstrate anti-apoptotic activity. Here we investigated the response of the wtNIH3T3, SPIβ and SPIα cells upon induction of apoptosis by UV radiation or incubation with TNFa . Apoptosis was analyzed by quantifying the number of blebbing cells, by FACS analysis of fragmenting DNA and by microscopic analysis of DAPI-stained nuclei.

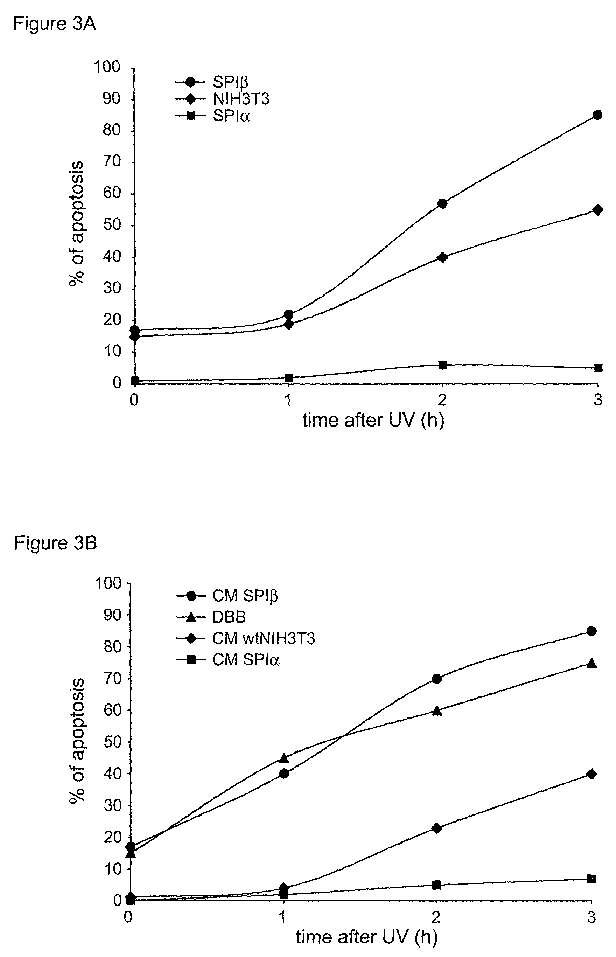

UV radiation (200 J/m²) induced apoptosis in wtNIH3T3, SPIβ and SPIα cells. However, significant differences in response to UV radiation were observed between the three cell lines: three hours after UV-radiation the SPIα cells showed a very low response (3% apoptotic) while the wtNIH3T3 and SPIβ cells were much more sensitive (55 and 85% apoptotic, respectively) and showed a high number of apoptotic cells. (Fig. 3A). From this observation it can be concluded that SPIα cells have acquired a resistance/defense against apoptosis whereas SPIβ cells have become more sensitive.

We investigated whether conditioned medium of SPIα SPIβ and wtNIH3T3 cells is able to protect SPIβ cells against UV-induced apoptosis. SPIβ cells were incubated for 4 h with conditioned medium before UV radiation and apoptosis was determined by counting the blebbing cells. Conditioned medium of SPIα cells was able to fully protect SPIβ cells against UV-induced apoptosis (Fig 3B). The conditioned medium of NIH3T3 cells was much less protective whereas the conditioned medium of SPIβ cells had no effect. Neutral lipid extracts of the various conditioned mediums gave similar results (data not shown).

The conditioned medium of SPIα cells was added to human neutrophils and the apoptosis as measured by FACS analysis after 24 h amounted to 20% as compared to 67% in the presence of control medium (DBB) After 48 h 60% of the neutrophils were protected against apoptosis by the SPIα medium.

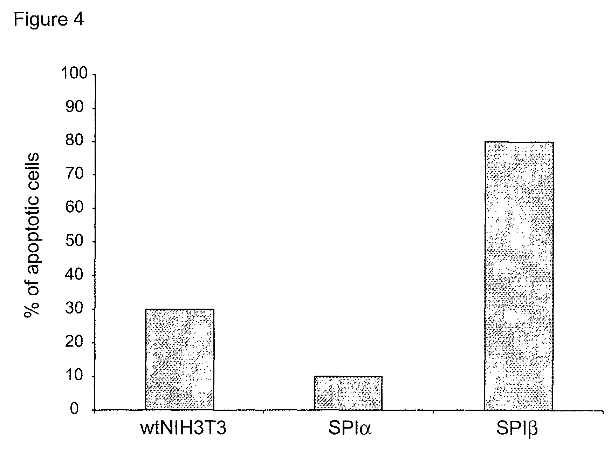

The conditioned medium of SPIα cells prepared in the presence of 10 µM indomethacin or 50µM NS398 had no effect on the survival of SPIβ cells upon UV-induced apoptosis.In addition to counting blebbing cells as a method to quantify apoptosis, we have analyzed DNA fragmentation by FACS analysis of cells that were labeled with propidium iodide. The FACS analysis confirmed the results that were obtained by scoring the blebbing, apoptotic cells. The amount of fragmented DNA 2 h after UV-radiation is low in SPIα cells, three times higher in wtNIH3T3 cells and eight times higher in SPIβ cells (Fig 4). When wtNIH3T3 or SPIβ cells were incubated for 4h withconditioned medium of SPIαcells before UV-treatment the level of fragmented DNA significantly decreased. On the other hand when these cells were incubated with conditioned medium of SPIα cells that was prepared in the presence of NS398, the anti-apoptotic activity was not observed (data not shown).

### COX-2 expression in wtNIH3T3, SPIα and SPIβ cells.

The finding that NS398 inhibits the production of the mitotic as well as of the anti-apoptotic activity indicates the involvement of COX-2 in the production process. We investigated the expression of COX-2 in wtNIH3T3, SPIβ and

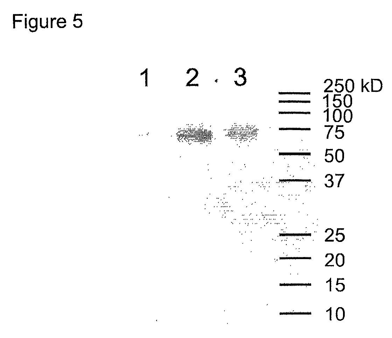

SPIα cells by western blotting. The expression of COX-2 is three-fold increased in SPIα cells and decreased in SPIβ cells as compared to the levels in wtNIH3T3 cells (fig. 5).

### LEGENDS TO FIGURES:

*Figure 1*: Cell proliferation as measured by [³H]-thymidine incorporation.
A. Effect of conditioned medium (CM) of wtNIH3T3 and SPIα cells on the growth of wtNIH3T3 cells. DBB is the control medium
B. Effect of the neutral lipid extracts of the conditioned medium of wtNIH3T3 and SPIα cells on the growth of wtNIH3T3 cells.
C. Effect of the COX-1/COX-2 inhibitor indomethacin on the production of the mitogenic factor produced by wtNIH3T3 and SPIα cells. The media were tested on wtNIH3T3 cells. White bars represent media without indomethacin treatment, gray bars represent media with indomethacin treatment.

Figure 2: Analysis of [¹⁴C]-arachidonic acid metabolites in conditioned medium of wtNIH3T3 and SPIα cells prepared in the absence or presence of NS398. Neutral lipids were extracted by ethylacetate, separated by thin layer chromatography and scanned for radioactivity. The gray peaks represent the metabolites produced as a result of PI-TPα overexpression (SPIα cells).
*Figure 3*: UV-induced apoptosis as estimated by counting blebbing cells.
A. Cells were submitted to UV-irradiation (200 J/m²) and the apoptosis was measured as a function of time after UV-treatment.
B. Effect of conditioned medium of wtNIH3T3, SPIα and SPIβ cells on the survival of SPIβ cells after UV-induced apoptosis. Conditioned medium was added to the SPIβ cells for four hours, after removal of the medium, the cells were treated with 200 J/m² UV and subsequently incubated with DBB for up to three hours and the apoptosis was estimated.

*Figure 4*: UV-induced apoptosis of wtNIH3T3, SPIα and SPIβ cells as measured by FACS analysis.
*Figure 5*: Western blot of cell lysates from wtNIH3T3 (lane 1), SPIα (lane 2) and SPIβ cells (lane 3) using anti-COX-2 antibody.

**Table 1**

| | SPIα | SPIβ | W†NIH3T3 |
|---|---|---|---|
| Fold increase in protein level | 2-3 | 15 | - |
| | | | |
| Cell cycle duration (h) | 13 | 35 | 21 |
| | | | |
| Cell density at confluency (×10⁵/cm²) | 0.53 | 0.14 | 0.20 |
| | | | |
| Changes in lipid metabolism | PI-PLA₂ activation | Increase in intracellular SM traffic | - |

### References

Avis, I., S. H. Hong, et al. (2001). "Five-lipoxygenase inhibitors can mediate apoptosis in human breast cancer cell lines through complex eicosanoid interactions." Faseb J 15(11): 2007-9.

Cockcroft, S. (2001). "Phosphatidylinositol transfer proteins couple lipid transport to phosphoinositide synthesis." Semin Cell Dev Biol 12(2): 183-91.

Dang, C. T., C. L. Shapiro, et al. (2002). "Potential role of selective COX-2 inhibitors in cancer management." Oncology (Huntingt) 16(5 Suppl 4): 30-6.

Engidawork, E., T. Gulesserian, et al. (2001). "Alteration of caspases and apoptosis-related proteins in brains of patients with Alzheimer's disease." Biochem Biophys Res Commun 281(1): 84-93.

Geijtenbeek, T. B., de Groot, E., van Baal, J., Brunink, F., Westerman, J., Snoek, G.T., Wirtz, K.W.A. (1994). "Characterization of mouse phosphatidylinositol transfer protein expressed in Escherichia coli." Biochim. Biophys. Acta 1213(3): 309-318.

Graham, A. M., M. M. Dollinger, et al. (1998). "Bile duct cells in primary biliary cirrhosis are 'primed' for apoptosis." Eur J Gastroenterol Hepatol 10(7): 553-7.

Harris, A., H. S. Chung, et al. (1999). "Progress in measurement of ocular blood flow and relevance to our understanding of glaucoma and age-related macular degeneration." Prog Retin Eye Res 18(5): 669-87.

Lukiw, W. J. and N. G. Bazan (2000). "Neuroinflammatory signaling upregulation in Alzheimer's disease." Neurochem Res 25(9-10): 1173-84.

Macchia, L., M. Hamberg, et al. (1995). "Arachidonic acid metabolism in the human mast cell line HMC-1: 5-lipoxygenase gene expression and biosynthesis of thromboxane." Biochim Biophys Acta 1257(1): 58-74.

Morita, H., K. Takeda, et al. (1999). "Immunosuppressive effect of leukotriene B(4) receptor antagonist in vitro." Biochem Biophys Res Commun 264(2): 321-6.

Nagatsu, T. (2002). "Parkinson's disease: changes in apoptosis-related factors suggesting possible gene therapy." J Neural Transm 109(5-6): 731-45.

Ratnasinghe, D., P. J. Daschner, et al. (2001). "Cyclooxygenase-2, P-glycoprotein-170 and drug resistance; is chemoprevention against multidrug resistance possible?" Anticancer Res 21(3C): 2141-7.

Sabbah, H. N. and V. G. Sharov (1998). "Apoptosis in heart failure." Prog Cardiovasc Dis 40(6): 549-62.

Sathasivam, S., P. G. Ince, et al. (2001). "Apoptosis in amyotrophic lateral sclerosis: a review of the evidence." Neuropathol Appl Neurobiol 27(4): 257-74.

Slice, L. W., L. Bui, et al. (2000). "Differential regulation of COX-2 transcription by Ras- and Rho-family of GTPases." Biochem Biophys Res Commun 276(2): 406-10.

Smith, W. L. and R. Langenbach (2001). "Why there are two cyclooxygenase isozymes." J Clin Invest 107(12): 1491-5.

Snoek, G. T., Berrie, C.P., Geijtenbeek, T.B.H., van der Helm, H.A., Cadee, J.A., Iurisci, C., Corda, D., Wirtz, K.W.A. (1999). "Overexpression of phosphatidylinositol transfer protein alpha in NIH3T3 cells activates a phospholipase A." J. Biol. Chem. 274: 35393-35399.

Tai, H.-H., C. L. Tai, et al. (1979). "Biosynthesis of Prostaglandins in Rabbit Kidney Medulla." Biochem. J. 154: 257-264.

van Tiel, C. M., Luberto, C., Snoek, G.T., Hannun, Y.A., Wirtz, K.W.A. (2000). "Rapid replenishment of sphingomyelin in the plasma membrane upon degradation by sphingomyelinase in NIH3T3 cells overexpressing the phosphatidylinositol transfer protein beta." Biochem. J. 346(2): 537-543.

Vane, J. R., Y. S. Bakhle, et al. (1998). "Cyclooxygenases 1 and 2." Annu Rev Pharmacol Toxicol 38: 97-120.

Vane, J. R. and R. M. Botting (1998). "Anti-inflammatory drugs and their mechanism of action." Inflamm Res 47 Suppl 2: S78-87.

Williams, C. S., M. Mann, et al. (1999). "The role of cyclooxygenases in inflammation, cancer, and development." Oncogene 18(55): 7908-16.

Wirtz, K. W. A. (1997). "Phospholipid transfer proteins revisited." Biochem. J. 324(2): 353-360.

## Claims

1. An isolated supernatant or derivative thereof, of a cell having an active COX system, said cell over-expressing PI-TPα.

2. An isolated or recombinant arachidonic acid metabolite, or a functional equivalent thereof, extractable in the organic phase and capable of increasing the survival time of at least 50 % of isolated human neutrophilic granulocytes to at least 48 hours, capable of increasing the mitotic activity of quiescent NIH3T3 cells and capable of at least in part protecting quiescent NIH3T3 and SPIβ cells against induced apoptosis.

3. An isolated or recombinant arachidonic acid metabolite, or a functional equivalent thereof, according to claim 2, obtainable from a supernatant according to claim 1.

4. An arachidonic acid metabolite, or a functional equivalent thereof, according to claims 2 or 3, which is, when using an organic solvent, detectable in thin layer chromatography at a position different from that of prostaglandins PGF_{2α} and PGE₂.

5. A composition comprising an arachidonic acid metabolite, or a functional equivalent thereof, according to any of claims 2 to 4, further comprising at least one other arachidonic acid metabolite.

6. A composition comprising an arachidonic acid metabolite, or a functional equivalent thereof, according to any of claims 2 to 4, further comprising a further arachidonic acid metabolite obtainable from a supernatant according to claim 1

7. A method of obtaining an arachidonic acid metabolite according to any of claims 2 to 4 comprising:
a increasing PI-TPα expression in cells comprising COX,
b culturing said cells in vitro,
c collecting supernatant medium of said cells, said cells or a combination thereof, wherein optionally the cells are treated with a calcium ionophore prior to said collection,
d and extracting eicosanoids from said supernatant medium, said cells or a combination thereof.

8. A method according to claim 7, further comprising extracting eicosanoids by ethyl acetate and/or a functional equivalent thereof.

9. A method according to claim 7 or 8, further comprising at least partially separating said arachidonic acid metabolite from prostaglandins PGF_{2α} and PGE₂.

10. An arachidonic acid metabolite obtainable by methods according to any of claims 7-9.

11. Use of a cell supernatant according to claim 1 and/or an arachidonic acid metabolite according to any of claims 2 to 4 and/or a functional equivalent thereof or a composition according to claim 5 or claim 6, for at least in part inhibiting apoptosis of a eukaryotic cell.

12. Use of a cell supernatant according to claim 1 and/or an arachidonic acid metabolite according to any of claims 2 to 4 and/or a functional equivalent thereof or a composition according to claim 5 or claim 6, for the manufacture of a medicament for the treatment of apoptosis-related disease.

13. A pharmaceutical composition comprising a cell supernatant according to claim 1 and/or an arachidonic acid metabolite according to any of claims 2 to 4 and/or a functional equivalent thereof or a composition according to claim 5 or claim 6, and a suitable carrier or solvent.

14. A pharmaceutical composition according to claim 13 for use in the treatment of an apoptosis-related disease.

15. An isolated or recombinant antibody capable of specifically binding an arachidonic acid metabolite or a functional part, derivative and/or analogue according to any of claims 2 to 4, or a functional part, derivative and/or analogue of said antibody.

16. A method for identifying a cell carrying a functional receptor for an arachidonic acid metabolite, said receptor specifically increasing mitosis and decreasing apoptosis in said cell.

17. A method for identifying a functional receptor for an arachidonic acid metabolite, said receptor specifically increasing mitosis and decreasing apoptosis in said cell.

18. A cellular receptor for an arachidonic acid metabolite specifically increasing mitosis and decreasing apoptosis in a cell.

19. An antagonist to a cellular receptor according to claim 18, said antagonist capable of at least in part inhibiting the bioactivity of said arachidonic metabolite on its cellular receptor

20. A method for at least in part inhibiting the mitogenic and/or anti-apoptotic signalling activity of an arachidonic acid metabolite according to any of claims 2 to 4 and/or a functional equivalent thereof or a composition according to claim 5 or claim 6, comprising providing an antagonist of said arachidonic acid metabolite thereby at least in part inhibiting the bioactivity of said arachidonic metabolite on its cellular receptor.

21. A method according to claim 20 further comprising inhibiting COX enzymes.

22. A method according to any of claims 20 or 21 wherein said antagonist decreases the bio-availability of said arachidonic acid metabolite

23. A method according to any of claims 20 to 22 comprising providing an antibody according to claim 15.

24. Use of an antagonist according to claim 19 for the production of a medicament for the treatment of cancer.

25. Use according to claim 24 also comprising the use of COX-inhibitor.

26. A pharmaceutical composition comprising an antagonist according to claim 19 and/or a COX-inhibitor and a suitable carrier or solvent.

27. Use of a pharmaceutical composition according to claim 26 for the preparation of a medicament for the treatment of cancer.
